(19) Europäisches Patentamt
European Patent Office
Office européen des brevets

(11) EP 1 048 648 A1

(12) EUROPÄISCHE PATENTANMELDUNG

(43) Veröffentlichungstag:
**02.11.2000 Patentblatt 2000/44**

(51) Int. Cl.[7]: **C07C 315/06**, C07C 317/04, H01M 6/16, H01M 10/40

(21) Anmeldenummer: **00108129.8**

(22) Anmeldetag: **13.04.2000**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **28.04.1999 DE 19919346**

(71) Anmelder: **MERCK PATENT GmbH**
**64293 Darmstadt (DE)**

(72) Erfinder:
- **Ignatiev, Nikolai, Dr.**
  **47058 Duisburg (DE)**
- **Sartori, Peter, Prof. Dr.**
  **47495 Rheinberg (DE)**
- **Barthen, Peter**
  **47137 Duisburg (DE)**

(54) **Verfahren zur Aufreinigung von Methanid-Elektrolyten (I)**

(57) Die Erfindung betrifft ein Verfahren zur Darstellung von organischen Methanid-Elektrolyten der Formule (I) $MC(SO_2(C_xF_{2x+1}))_3$, in verwendungsfähiger Qualität für Anwendung in elektrochemischen Zellen und Batterien.

Das Verfahren umfaßt die folgenden Schritte:

(i) Umsetzung eines Methanides der Formel (I) mit konzentrierter Schwefelsäure und fraktionierter Rektifikation der gebildeten freien Säure des besagten Methanides,

(ii) Umsetzung des aus (i) gewonnenen Produktes der Formel (I) mit M=H mit Phosphorpentoxid oberhalb des Schmelzpunktes und anschließend fraktionierter Rektifikation,

(iii) Aufnehmen des Produktes aus (ii) in einem aprotischen organischen Lösungsmittel und Umsetzung mit metallischem Li, Na, K, Rb, Cs, Mg, Ca, Sr or Ba oder deren Chloriden oder Hydriden, für M=Li auch mit Alkyllithium, zu den entsprechenden Metall-Methaniden der Formel (I) und ggf. Entfernen von überschüssigem Reagenz.

EP 1 048 648 A1

## Beschreibung

Die Erfindung betrifft ein Verfahren zur Darstellung von organischen Methanid-Elektrolyten in verwendungsfähiger Qualität zur Anwendung in elektrochemischen Zellen.

Die Verbindungsklasse der Tris(perfluoralkansulfonyl)methanide wurde erstmalig von Turowsky et. al. in Inorgan. Chem., 1988, 27, 2135-2137 anhand des Tris(trifluormethansulfonyl)methans beschrieben. Diese C-H acide Verbindung reagiert mit Basen zu den entsprechenden Salzen. Das Anion liegt planar vor, wobei die negative Ladung durch die stark elektronenziehenden Substituenten sehr gut delokalisiert werden kann.

Das Lithiumsalz Lithium[tris(trifluormethansulfonyl)methanid] wird aufgrund der hohen Leitfähigkeit und guten Löslichkeit in aprotischen Lösungsmitteln seit längerem bezüglich seiner Eignung als Leitsalz in Sekundärbatterien untersucht. Weitere Vorteile dieses Salzes sind die hohe elektrochemische und thermische Stabilität.

Zur Darstellung solcher Verbindungen gibt es zwei Verfahren. Nach Turowsky et. al. wird Tris[trifluormethansulfonyl]methan durch Grignard-Reaktion mit Trifluormethansulfonylfluorid dargestellt.

Ein zweistufiges Verfahren nach Koshar et. al. in J. Org. Chem., 1973, 38, 3358-3363 und Benrabah et.al. in J. Chem. Soc. Faraday Trans., 1993, 89(2), 355-359 führt ebenfalls zu Tris[trifluormethansulfonyl]methan.

Beide Verfahren führen zu Produkten, die zur Verwendung als Leitsalz einer Aufreinigung bedürfen. Bisher verwendete Reinigungsverfahren, welche auf Umsetzung des solvatisierten Salzes mit Aktivkohle und Umkristallisation beruhen, führen zu Produkten mit einer Reinheit von in der Regel nicht mehr als 99,5% und weisen noch störende Verunreinigungen von Wasser und Fremdionen auf. Salze dieser Qualität sind jedoch für den Einsatz in organischen Elektrolyten nicht geeignet.

Aufgabe der vorliegenden Erfindung ist es deshalb, ein preiswertes, einfach durchführbares Verfahren zur Verfügung zu stellen, durch das organische Methanid-Elektrolyte in hochreiner Form erhalten werden, so daß die hergestellten Produkte für die Anwendung in Batterieelektrolyten geeignet sind. Als hochrein werden erfindungsgemäß Reinheitsgrade mit mehr als 99,5% bezeichnet.

Die erfindungsgemäße Aufgabe wird gelöst durch ein Verfahren zur Herstellung hochreiner als Elektrolyte geeigneter Methanide der Formel

$$MC(SO_2\,(C_xF2_{x+1}))_3 \qquad (I)$$

worin

x 1, 2, 3, 4, 5, 6, 7 oder 8 und

M H, Li, Na, K, Rb, Cs, $Mg_{1/2}$, $Ca_{1/2}$, $Sr_{1/2}$ oder $Ba_{1/2}$ bedeutet,

durch Aufreinigung, dadurch gekennzeichnet, daß es folgende Schritte umfaßt:

(i) Umsetzung eines Methanides der Formel (I) mit konzentrierter Schwefelsäure und fraktionierter Rektifikation der gebildeten freien Säure des besagten Methanides,

(ii) Umsetzung des aus (i) gewonnenen Produktes der Formel (I) mit M=H mit Phosphorpentoxid oberhalb des Schmelzpunktes und anschließende fraktionierte Rektifikation,

(iii) Aufnehmen des Produktes der Formel (I) mit M=H aus (ii) in einem aprotischen organischen Lösungsmittel und Umsetzung mit metallischem Li, Na, K, Rb, Cs, Mg, Ca, Sr oder Ba oder deren Chloriden oder Hydriden oder mit Alkyllithium zu den entsprechenden Metall-Methaniden nach Formel (I) und ggf. Entfernen von überschüssigem Reagenz.

Das erfindungsgemäße Verfahren liefert Materialien mit einem Reinheitsgehalt von mehr als 99,5%, vorzugsweise zwischen 99,6% und 99,9%, die somit für den Einsatz als Elektrolyten in Batterien geeignet sind.

Überraschend wurde dabei gefunden, daß durch die Umsetzung mit konzentrierter Schwefelsäure das Ausgangsmaterial stabilisiert wird und somit zersetzungsfrei destilliert werden kann. Außerdem kann durch die Zugabe äquivalenter Mengen oder einem Überschuß an konzentrierter Schwefelsäure die freie Säure $HC(SO_2CF_3)_3$ direkt aus ihren Salzen gewonnen und gereinigt werden.

Durch die erfindungsgemäße Zugabe der stark hygroskopischen Schwefelsäure wird bereits ein guter Trocknungseffekt erreicht, welcher durch Zugabe von Schwefeltrioxid, entsprechend dem Wassergehalt des Rohproduktes, noch gesteigert werden kann.

Es wurde auch gefunden, daß die fraktionierte Rektifikation der Reinfraktion unter Zusatz von Phosphorpentoxid zu einem Reinprodukt mit einem Wassergehalt zwischen 5 und 30 ppm, vorzugsweise zwischen 10 und 20 ppm führt. Diese hocheffektive Trocknung kann dabei ökonomisch an beliebig großen Mengen vorgenommen werden.

Besonders vorteilhaft ist die erfindungsgemäße Verwendung eines Lösungsmittels, welches ausschließlich oder anteilig im fertigen Elektrolyten verwendet wird. Dadurch kann auf die aufwendige Isolierung des Salzes verzichtet werden.

Bei der Umsetzung nach Verfahrensschritt (iii) entsteht als Nebenprodukt gasförmiger Wasserstoff, Chlorwasserstoff oder Alkane, die problemlos entfernt werden können. Bei dieser Reaktion wurde festgestellt, daß die erfindungsgemäße Neutralisation von $HC(SO_2CF_3)_3$ Zersetzungserscheinungen am Anion vermeidet, wie sie bei üblichen Verfahren beobachtet wurden.

Die erfindungsgemäße Reduktion des Volumens des Elektrolyten durch Destillation hat den entscheidenden Vorteil, daß durch den in der Lösung vorhandenen großen Überschuß an erwünschtem Sauerstoffnukleophil unerwünschte Nukleophile aus der Koordinationssphäre des Lithiums verdrängt werden. Dieser Effekt erlaubt ein Abdestillieren von Verunreinigungen. Man erhält einen hochkonzentrierten Elektrolyten, welcher geringe Lager- und Transportkosten ermöglicht.

Es wurde gefunden, daß die Verdünnung des hochkonzentrierten Elektrolyten mit frei wählbaren Lösungsmitteln erfolgen kann. Daher ist es auf einfache Weise möglich, das optimale Lösungsmittelgemisch einzusetzen und Elektrolyte beliebiger Konzentration bereitzustellen.

Die Aufreinigung besteht im wesentlichen aus 3 Verfahrensschritten, an die sich vorzugsweise noch zwei weitere Schritte anschließen können.

1. Schritt:

Ein Methanid der Formel (I), welches eine Reinheit zwischen 90% und 99,5% aufweist, wird chargenweise in konzentrierte Schwefelsäure (96%-98%-ige Schwefelsäure) gegeben und bei Temperaturen zwischen 10 und 40°C gerührt. Bevorzugt wird bei Temperaturen zwischen 20 und 30°C mit frisch destillierter Schwefelsäure umgesetzt. Die Schwefelsäure wird in äquivalenten Mengen oder im Überschuß zugegeben. Die Rektifikationsapparatur mit isothermer Kolonne wird unter Schutzgasatmosphäre ausgeheizt. Die Destillationsbrücke muß durch Heizbänder o.ä. beheizbar sein. Damit wird die Destillationsbrücke auf einer konstanten Temperatur oberhalb des jeweiligen Schmelzpunktes gehalten. In dieser Apparatur wird fraktioniert rektifiziert.

2. Schritt:

Die Reinfraktion aus Schritt 1 wird in einer unter Schutzgasatmosphäre ausgeheizten Destillationsapparatur mit Phosphorpentoxid versetzt. Das Gemisch wird bei Temperaturen am und oberhalb des Schmelzpunktes der Säure 15 min bis 5 Stunden gerührt. Die Umsetzung erfolgt bevorzugt zwischen 30 min und 90 min. Das Gemisch wird anschließend unter Vakuum fraktioniert rektifiziert.

Die fraktionierte Rektifikation der Reinfraktion aus Schritt 1 unter Zusatz von Phosphorpentoxid führt zu einem Reinprodukt mit einem Wassergehalt von vorzugsweise 10 bis 30 ppm.

3. Schritt:

Das Produkt aus Schrift 2 wird unter trockener Inertgasatmosphäre in polaren organische Lösungsmittel gelöst. Besonders geeignet sind aprotischen Lösungsmitteln wie DMC, DEC, EC, PC, BC, VC, Cyclopentanon, Sulfolan, DMS, 3-Methyl-1,3-oxazolidin-2-on, $\gamma$-Butyrolacton, EMC, MPC, BMC, EPC, BEC, DPC, 1,2-Diethoxymethan, THF, 2-Methyltetrahydrofuran, 1,3-Dioxolan, Methylacetat, Ethylacetat und deren Mischungen. Die Lösung wird mit Lithiumhydrid, mit metallischem Lithium (Li), mit Lithiumchlorid, in situ durch Anwendung einer Lithiumanode oder mit Alkyllithium versetzt. Zur Darstellung der Natrium-, Kalium-, Rubidium-, Cäsium-, Magnesium-, Calcium-, Strontium- oder Bariumverbindungen kann mit metallischem Natrium (Na), Kalium (K), Rubidium (Rb), Cäsium (Cs), Magnesium (Mg), Calcium (Ca), Strontium (Sr) oder Barium (Ba), Natrium-, Kalium-, Rubidium-, Cäsium-, Magnesium-, Calcium-, Strontium- oder Bariumchlorid, Natrium-, Kalium-, Rubidium-, Cäsium-, Magnesium-, Calcium-, Strontium- oder Bariumhydrid umgesetzt werden. Das Gemisch wird 10 min bis 24 Stunden bei Temperaturen zwischen 10°C und 200°C gerührt. Bevorzugt wird 25 min bis 5 Stunden bei Temperaturen zwischen 20°C und 100°C umgesetzt. Anschließend wird überschüssiges Alkali- oder Erdalkalireagenz abfiltriert.

4. Schritt:

Das Volumen der Lösung von Schritt 3 wird bei Bedarf auf 2/3 bis 1/4 reduziert. Vorzugsweise wird das Lösungsmittel auf 1/3 reduziert. Die Destillation erfolgt bei Normaldruck am Siedepunkt des entsprechenden Lösungsmittels. Die Destillation kann auch unter Vakuum erfolgen. Die Siedepunkte verschieben sich entsprechend.

5. Schritt:

Der hochviskose Elektrolyt kann mit beliebigen Lösungsmitteln und Lösungsmittelgemischen beliebig stark verdünnt werden. Geeignet sind alle Lösungsmittel und -gemische die in elektrochemischen Speichermedien eingesetzt werden. Die Zusammensetzung des Elektrolyten kann somit entsprechend den speziellen Anforderungen angepaßt werden.

Das preiswerte und mit einfachen Mitteln und Apparaturen durchführbare Verfahren liefert Produkte in einer Qualität, die für den Einsatz in Batterien geeignet sind, in guten Ausbeuten. Bei diesem Verfahren werden keine explosionsfähigen oder toxischen Nebenprodukte gebildet.

Ebenso können die Methanide in Anteilen zwischen 1 und 99% in Kombination mit anderen Leitsalzen, die in elektrochemischen Zellen Anwendung finden, verwendet werden. Geeignet sind z.B. Leitsalze ausgewählt aus der Gruppe $LiPF_6$, $LiBF_4$, $LiClO_4$, $LiAsF_6$, $LiCF_3SO_3$, $LiN(CF_3SO_2)_2$ oder $LiC(CF_3SO_2)_3$ und deren Mischungen. Die Elektrolyte können auch organische Isocyanate (DE 199 44 603) zur Herabsetzung des Wassergehaltes enthalten. Ebenso können die Elektrolyte organische Alkalisalze (DE 199 10 968) als Additiv enthalten. Geeignet sind Alkaliborate der allgemeinen Formel

$$Li^+ B^-(OR^1)_m(OR^2)_p$$

worin,

m und p 0, 1, 2, 3 oder 4 mit m+p=4 und
$R^1$ und $R^2$ gleich oder verschieden sind,

gegebenenfalls durch eine Einfach- oder Doppelbindung direkt miteinander verbunden sind,

jeweils einzeln oder gemeinsam die Bedeutung eines aromati schen oder aliphatischen Carbon-, Dicarbon- oder Sulfonsäurerestes haben, oder
jeweils einzeln oder gemeinsam die Bedeutung eines aromatischen Rings aus der Gruppe Phenyl, Naphthyl, Anthracenyl oder Phenanthrenyl, der unsubstituiert oder ein- bis vierfach durch A oder Hal substituiert sein kann, haben oder
jeweils einzeln oder gemeinsam die Bedeutung eines heterocyclischen aromatischen Rings aus der Gruppe Pyridyl, Pyrazyl oder Bipyridyl, der unsubstituiert oder ein- bis dreifach durch A oder Hal substituiert sein kann, haben oder
jeweils einzeln oder gemeinsam die Bedeutung einer aromatischen Hydroxysäure aus der Gruppe aromatischer Hydroxy-Carbonsäuren oder aromatischer Hydroxy-Sulfonsäuren, der unsubstituiert oder ein- bis vierfach durch A oder Hal substituiert sein kann,
haben und
Hal F, Cl oder Br
und
A Alkyl mit 1 bis 6 C-Atomen, das ein- bis dreifach halogeniert sein kann, bedeuten. Ebenso geeignet sind Alkalialkoholate der allgemeinen Formel

$$Li^+ OR^-$$

sind, worin R
die Bedeutung eines aromatischen oder aliphatischen Carbon-, Dicarbon- oder Sulfonsäurerestes hat, oder
die Bedeutung eines aromatischen Rings aus der Gruppe Phenyl, Naphthyl, Anthracenyl oder Phenanthrenyl, der unsubstituiert oder ein- bis vierfach durch A oder Hal substituiert sein kann, hat oder
die Bedeutung eines heterocyclischen aromatischen Rings aus der Gruppe Pyridyl, Pyrazyl oder Bipyridyl, der unsubstituiert oder ein- bis dreifach durch A oder Hal substituiert sein kann, hat oder
die Bedeutung einer aromatischen Hydroxysäure aus der Gruppe aromatischer Hydroxy-Carbonsäuren oder aromatischer Hydroxy-Sulfonsäuren, der unsubstituiert oder ein- bis vierfach durch A oder Hal substituiert sein kann, hat und
Hal F, Cl, oder Br,
und
A Alkyl mit 1 bis 6 C-Atomen, das ein- bis dreifach halogeniert sein kann.

Auch Lithiumkomplexsalze der Formel

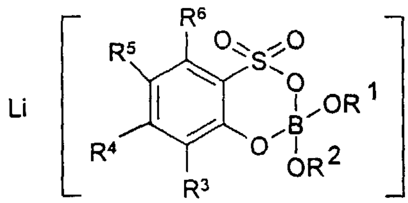

wobei

$R^1$ und $R^2$ gleich oder verschieden sind, gegebenenfalls durch eine Einfach- oder Doppelbildung direkt miteinander verbunden sind, jeweils einzeln oder gemeinsam die Bedeutung eines aromatischen Rings aus der Gruppe Phenyl, Naphthyl, Anthracenyl oder Phenanthrenyl, der unsubstituiert oder ein- bis sechsfach durch Alkyl ($C_1$ bis $C_6$), Alkoxygruppen ($C_1$ bis $C_6$) oder Halogen (F, Cl, Br) substituiert sein kann, haben,

oder jeweils einzeln oder gemeinsam die Bedeutung eines aromatischen heterozyklischen Rings aus der Gruppe Pyridyl, Pyrazyl oder Pyrimidyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann, haben,

oder jeweils einzeln oder gemeinsam die Bedeutung eines aromatischen Rings aus der Gruppe Hydroxylbenzoecarboxyl, Hydroxylnaphthalincarboxyl, Hydroxylbenzoesulfonyl und Hydroxylnaphthalinsulfonyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann, haben,

$R^3$-$R^6$ können jeweils einzeln oder paarweise, gegebenenfalls durch eine Einfach oder Doppelbindung direkt miteinander verbunden, folgende Bedeutung haben:

1. Alkyl ($C_1$ bis $C_6$),Alkyloxy ($C_1$ bis $C_6$) oder Halogen (F, Cl, Br)

2. ein aromatischer Ring aus den Gruppen

Phenyl, Naphthyl, Anthracenyl oder Phenanthrenyl, der unsubstituiert oder ein- bis sechsfach durch Alkyl ($C_1$ bis $C_6$), Alkoxygruppen ($C_1$ bis $C_6$) oder Halogen (F, Cl, Br) substituiert sein kann,

Pyridyl, Pyrazyl oder Pyrimidyl, der unsubstituiert oder ein- bis vierfach durch Alkyl ($C_1$ bis $C_6$), Alkoxygruppen ($C_1$ bis $C_6$) oder Halogen (F, Cl, Br) substituiert sein kann,

die über folgendes Verfahren (DE 199 32 317) dargestellt werden

a) 3-, 4-, 5-, 6-substituiertes Phenol in einem geeigneten Lösungsmittel mit Chlorsulfonsäure versetzt wird,
b) das Zwischenprodukt aus a) mit Chlortrimethylsilan umgesetzt, filtriert und fraktioniert destilliert wird,
c) das Zwischenprodukt aus b) mit Lithiumtetramethanolat-borat(1-), in einem geeigneten Lösungsmittel umgesetzt und daraus das Endprodukt isoliert wird, können im Elektrolyten enthalten sein.

Ebenso können die Elektrolyte Verbindungen der folgenden Formel (DE 199 41 566)

$$[([R^1(CR^2R^3)_k]_lA_x)_yKt]^+ \ ^-N(CF_3)_2$$

wobei

Kt= N, P, As, Sb, S, Se

A= N, P, P(O), O, S, S(O), $SO_2$, As, As(O), Sb, Sb(O)

$R^1$, $R^2$ und $R^3$
gleich oder verschieden

H, Halogen, substituiertes und/oder unsubstituiertes Alkyl $C_nH_{2n+1}$, substituiertes und/oder unsubstituiertes Alkenyl mit 1-18 Kohlenstoffatomen und einer oder mehreren Doppelbindungen, substituiertes und/oder unsubstituiertes Alkinyl mit 1-18 Kohlenstoffatomen und einer oder mehreren Dreifachbindungen, substituiertes und/oder unsubstituiertes Cycloalkyl $C_mH_{2m-1}$, ein- oder mehrfach substituiertes und/oder unsubstituiertes Phenyl, substituiertes und/oder unsubstituiertes Heteroaryl,

A kann in verschiedenen Stellungen in $R^1$, $R^2$ und/oder $R^3$ eingeschlossen sein,

Kt kann in cyclischen oder heterocyclischen Ring eingeschlossen sein,

die an Kt gebundenen Gruppen können gleich oder verschieden sein
mit

n= 1-18

m= 3-7

k= 0, 1-6

l= 1 oder 2 im Fall von x=1 und 1 im Fall x=0

x= 0,1

y= 1-4
bedeuten, enthalten. Das Verfahren zur Herstellung dieser Verbindungen ist dadurch gekennzeichnet, daß ein Alkalisalz der allgemeinen Formel

$$D^{+}\ {}^{-}N(CF_3)_2 \qquad (II)$$

mit $D^+$ ausgewählt aus der Gruppe der Alkalimetalle in einem polaren organischen Lösungsmittel mit einem Salz der allgemeinen Formel

$$[(\{R^1(CR^2R^3)_k\}_lA_x)_yKt]^+\ {}^-E \qquad (III)$$

wobei

Kt, A, $R^1$, $R^2$, $R^3$, k, l, x und y die oben angegebene Bedeutung haben und

$^-E$ $F^-$, $Cl^-$, $Br^-$, $I^-$, $BF_4^-$, $ClO_4^-$, $AsF_6^-$, $SbF_6^-$ oder $PF_6^-$
bedeutet, umgesetzt wird.

Aber auch Elektrolyte enthaltend Verbindungen der allgemeinen Formel (DE 199 53 638)

$$X\text{-}(CYZ)_m\text{-}SO_2N(CR^1R^2R^3)_2$$

mit

X H, F, Cl, $C_nF_{2n+1}$, $C_nF_{2n-1}$, $(SO_2)_kN(CR^1R^2R^3)_2$

Y H, F, Cl

Z H, F, Cl

$R^1$, $R^2$, $R^3$ H und/oder Alkyl, Fluoralkyl, Cycloalkyl

m 0-9 und falls X=H , m≠0

n 1-9

k 0, falls m=0 und k=1, falls m=1-9,
hergestellt durch die Umsetzung von teil- oder perfluorierten Alkysulfonylfluoriden mit Dimethylamin in organischen Lösungsmitteln sowie Komplexsalze der allgemeinen Formel (DE 199 51 804)

$$M^{x+}[EZ]^{y-}_{x/y}$$

worin bedeuten:

x, y 1, 2, 3, 4, 5, 6

$M^{x+}$ ein Metallion

E einer Lewis-Säure, ausgewählt aus der Gruppe $BR^1R^2R^3$, $AlR^1R^2R^3$, $PR^1R^2R^3R^4R^5$, $AsR^1R^2R^3R^4R^5$, $VR^1R^2R^3R^4R^5$,

$R^1$ bis $R^5$ gleich oder verschieden, gegebenenfalls durch eine Einfach- oder Doppelbildung direkt miteinander verbunden sind, jeweils einzeln oder gemeinsam die Bedeutung

eines Halogens (F, Cl, Br),

eines Alkyl- oder Alkoxyrestes ($C_1$ bis $C_8$) der teilweise oder vollständig durch F, Cl, Br substituiert sein kann,

eines, gegebenenfalls über Sauerstoff gebundenen aromatischen Rings aus der Gruppe Phenyl, Naphthyl, Anthracenyl oder Phenanthrenyl, der unsubstituiert oder ein- bis sechsfach durch Alkyl ($C_1$ bis $C_8$) oder F, Cl, Br substituiert sein kann

eines, gegebenenfalls über Sauerstoff gebundenen aromatischen heterozyklischen Rings aus der Gruppe Pyridyl, Pyrazyl oder Pyrimidyl, der unsubstituiert oder ein- bis vierfach durch Alkyl ($C_1$ bis $C_8$) oder F, Cl, Br substituiert sein kann, haben können und

Z $OR^6$, $NR^6R^7$, $CR^6R^7R^8$, $OSO_2R^6$, $N(SO_2R^6)(SO_2R^7)$, $C(SO_2R^6)(SO_2R^7)(SO_2R^8)$, $OCOR^6$, wobei

$R^6$ bis $R^8$ gleich oder verschieden sind, gegebenenfalls durch eine Einfach- oder Doppelbindung direkt miteinander verbunden sind, jeweils einzeln oder gemeinsam die Bedeutung

eines Wasserstoffs oder die Bedeutung wie $R^1$ bis $R^5$ haben, hergestellt durch Umsetzung von einem entsprechenden Bor- oder Phosphor-Lewis-Säure-Solvenz-Adukt mit einem Lithium- oder Tetraalkylammonium-Imid, -Methanid oder -Triflat, können verwendet werden.

Ebenso können sie in Mischungen eingesetzt werden die Boratsalze (DE 199 59 722) der allgemeinen Formel:

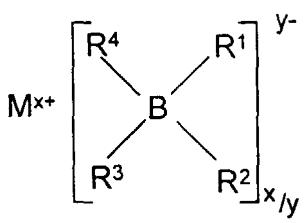

enthalten, worin bedeuten:

M ein Metallion oder Tetraalkylammoniumion

x,y 1, 2, 3, 4, 5 oder 6

$R^1$ bis $R^4$ gleich oder verschieden, gegebenenfalls durch eine Einfach- oder Doppelbindung direkt miteinander verbunder Alkoxy- oder Carboxyreste ($C_1$-$C_8$).

Diese Elektrolyte können in elektrochemischen Zellen eingesetzt werden, mit Kathoden aus gängigen Lithium-Interkalations und Insertionsverbindungen aber auch mit Kathodenmaterialien, die aus Lithium-Mischoxid-Partikel bestehen, die mit einem oder mehreren Metalloxiden oder Polymeren beschichtet sind.

Lithium-Mischoxid-Partikel beschichtet mit einem oder mehreren Metalloxiden erhalten durch ein Verfahren (DE 199 22 522) dadurch gekennzeichnet, daß die Partikel in einem organischen Lösungsmittel suspendiert werden, die Suspension mit einer Lösung einer hydrolysierbaren Metallverbindung und einer Hydrolyselösung versetzt und danach die beschichteten Partikel abfiltriert, getrocknet und gegebenenfalls calciniert werden. Lithium-Mischoxid-Partikel beschichtet mit einem oder mehreren Polymeren erhalten durch ein Verfahren (DE 199 46 066), dadurch gekennzeichnet, daß die Partikel in einer Lösung, enthaltend Polymere aus der Gruppe Polyimide, Polyaniline, Polypyrrole, Polythiophene, Polyacetylene, Polyacrylnitrile, carbonisierten Polyacrylnitrile, Poly-p-phenylene, Polyphenylenvinylene, Polyquinoline, Polyquinoxaline, Polyphtalocyaninsiloxane, Polyvinylidenfluoride, Polytetrafluorethylene, Polyethylmetacrylate, Polymethylmetacrylate, Polyamide, Copolymeren mit Vinylethern, Cellulose, Polyfluorethylene, Polyvinylalkohole und Polyvinylpyridine sowie deren Derivate ausgewählt, suspendiert werden und danach die beschichteten Partikel abfiltriert, getrocknet und gegebenenfalls calciniert werden.

Die nachfolgenden Beispiele sollen die Erfindung näher erläutern, ohne sie jedoch zu beschränken.

Beispiele

Beispiel 1

Aufreinigung von Tris(perfluoralkansulfonyl)methaniden

1.Schritt:

Lithium- bzw. Cäsium[tris(trifluormethansulfonyl)methanid (Reinheiten 97,5-99,5 %) wurde portionsweise in frisch destillierte Schwefelsäure gegeben und zehn Minuten bei Raumtemperatur gerührt. Anschließend wurde der Rundkolben an einer unter Argonatmosphäre ausgeheizten Destillationsapparatur mit isothermer Kolonne angesetzt und bei einem Druck von 10 Pa fraktioniert rektifiziert. Die Destillationsbrücke wurde ab dem Kolonnenkopf mit einem Heizband versehen um eine konstante Temperatur oberhalb des jeweiligen Schmelzpunktes in der Brücke zu erhalten. Die Schliffverbindungen wurden mit Teflonmanschetten versehen. In gleicher Weise wurde mit anderen Derivaten von Tris(perfluoralkansulfonyl)methanen verfahren. In Tabelle 1 sind die unterschiedlichen Methanide und die Ausbeuten an freier Säure angegeben.

Tabelle 1

| Verbindung | Menge [g] | Menge [mol] | Siedepunkt d. Säure [°C] [10Pa] | Säure Ausbeute [%] |
|---|---|---|---|---|
| $Li^+ C(CF_3SO_2)_3^-$ | 17 | 0,04 | 132-136 | 72 |
| $Cs^+ C(CF_3SO_2)_3^-$ | 27 | 0,05 | 135-136 | 68 |
| $Li^+ C(CF_3SO_2)_3^-$ | 68 | 0,16 | 135-137 | 87 |
| $Li^+ C(CF_3SO_2)_2(C_4F_9SO_2)^-$ | 19 | 0,03 | 143-145 | 43 |
| $HC(CF_3SO_2)_3$ Gehalt 89% | 323 | 0,67 | 135-137 | 95 |

2. Schritt:

Ein Teil der Reinfraktion von $HC(CF_3SO_2)_3$ aus Schrift 1 wurde mit Phosphorpentoxid versetzt und an die unter Argonatmosphäre ausgeheizte Destillationsapparatur angesetzt. Das Gemisch wurde geschmolzen, bei 90°C eine Stunde unter Normaldruck gerührt, und im Vakuum fraktioniert rektifiziert.

| | |
|---|---|
| Eingewogene Menge Tris(trifluormethansulfonyl)methan | 86 g |
| Zugegebene Menge Phosphorpentoxid | 4 g |
| Druck | 10 Pa |
| 1. Fraktion 135-137°C | 11 g |
| 2. Fraktion 138°C | 62,4 g |

3.Schritt:

62,4 g (0,151 mol) Tris(trifluormethansulfonyl)methan wurden unter trockener Inertgasatmosphäre in 100 ml Diethylcarbonat gelöst und mit 1,215 g Lithiumhydrid umgesetzt. Nach vollständiger Zugabe wurde der Elektrolyt 30 Minuten gerührt und anschließend überschüssiges Lithiumhydrid über einen Filter abgetrennt.

4. Schritt:

Das Volumen des Elektrolyten wurde bei Raumtemperatur und einem Druck von $10^{-5}$ Pa auf 30 ml reduziert.

5. Schritt:

Der nun hochviskose farblose Elektrolyt wurde mit 120 ml Lösungsmittelgemisch (Ethylencarbonat: Dimethylcarbonat 1:1) verdünnt, so daß 150 ml eines 1 molaren Elektrolyten erhalten wurden.
Eine 500 µl NMR-Probe des fertigen Elektrolyten wurde mit 250 µl $CD_3COCD_3$ versetzt und vermessen.

Bruker Advance DRX 500 NMR-Spektrometer:
470, 53 MHz, 35°C

$^{19}$F-NMR-Spektrum:
$\delta$= -76.488; s; $Li^+$ $C(CF_3SO_2)_3^-$

Auch bei größtmöglicher Verstärkung des Spektrums ließen sich keine $^{19}$F-haltigen Verunreinigungen nachweisen.
Ein Wassergehalt von 28 ppm wurde festgestellt.

**Patentansprüche**

**1.** Verfahren zur Herstellung hochreiner, als Elektrolyte geeigneter Methanide der Formel

$$MC(SO_2\ (C_xF_{2x+1}))_3 \qquad (I)$$

worin

x 1, 2, 3, 4, 5, 6, 7 oder 8 und

M H, Li, Na, K, Rb, Cs, $Mg_{1/2}$, $Ca_{1/2}$, $Sr_{1/2}$ oder $Ba_{1/2}$

bedeutet,
durch Aufreinigung dadurch gekennzeichnet, daß es folgende Schritte umfaßt:

(i) Umsetzung eines Methanides der Formel (I) mit konzentrierter Schwefelsäure und fraktionierte Rektifikation der gebildeten freien Säure des besagten Methanides,

(ii) Umsetzung des aus (i) gewonnenen Produktes der Formel (I) mit M=H mit Phosphorpentoxid oberhalb des Schmelzpunktes und anschließende fraktionierte Rektifikation,

(iii) Aufnehmen des Produktes aus (ii) in einem aprotischen organischen Lösungsmittel und Umsetzung mit metallischem Li, Na, K, Rb, Cs, Mg, Ca, Sr oder Ba oder deren Chloriden oder Hydriden, für M=Li auch mit Alkyllithium, zu den entsprechenden Metall-Methaniden der Formel (I) und ggf. Entfernen von überschüssigem Reagenz.

**2.** Verfahren nach Anspruch 1, dadurch gekennzeichnet, daß nach erfolgtem Verfahrensschritt (iii) aus Anspruch 1 1/3 bis 3/4 des Lösungsmittels abdestilliert wird.

**3.** Verfahren nach Anspruch 2, dadurch gekennzeichnet, daß nach erfolgtem Verfahrensschritt (iii) aus Anspruch 1 2/3 des Lösungsmittels abdestilliert wird.

**4.** Verfahren nach Anspruch 3, dadurch gekennzeichnet, daß die konzentrierte Lösung mit frei wählbaren, geeigneten Lösungsmitteln gebrauchsfertig verdünnt wird.

**5.** Verfahren nach einem der Ansprüche 1 bis 4, dadurch gekennzeichnet, daß ein Lösungsmittel verwendet wird, das ausschließlich oder anteilig im fertigen Elektrolyt zum Einsatz kommt.

**6.** Verfahren nach einem der Ansprüche 1 bis 5, dadurch gekennzeichnet, daß ein Methanid der Formel (I) in Schritt (i) eingesetzt wird, welches eine Reinheit zwischen 90% und 99,5% aufweist.

**7.** Verfahren zur Herstellung hochreiner Lithium-Methanide der Formel

$$LiC(SO_2(C_xF_{2x+1}))_3 \qquad (II)$$

durch Aufreinigung, dadurch gekennzeichnet, daß die Verfahrensschritte gemäß einem der Ansprüche 1 bis 6 durchgeführt werden.

**8.** Verfahren nach Anspruch 7, dadurch gekennzeichnet, daß Schritt (iii) mit Lithiumhydrid in Dimethylcarbonat durchgeführt wird.

**9.** Verwendung von Metall-Methaniden der Formel (I) hergestellt nach einem Verfahren der Ansprüche 1 bis 8 in elektrochemischen Zellen und primären und sekundären Batterien.

**10.** Verwendung von Metall-Methaniden der Formel (I) mit einem Reinheitsgrad von mehr als 99,5% und einem Wassergehalt von <60 ppm als Elektrolyt in elektrochemischen Zellen und primären und sekundären Batterien.

Europäisches Patentamt

# EUROPÄISCHER RECHERCHENBERICHT

Nummer der Anmeldung
EP 00 10 8129

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| D,A | D. BENREBAH, ET AL.: "Comparative electrochemical study of new poly(oxyethylene)-Li salt complexes" JOURNAL OF THE CHEMICAL SOCIETY. FARADAY TRANSACTIONS., Bd. 89, Nr. 2, 21. Januar 1993 (1993-01-21), Seiten 355-359, XP002144674 Royal Society of Chemistry, Letchworth, GB ISSN: 0956-5000 * Seite 355, linke Spalte, letzter Absatz - Seite 356, linke Spalte * | 1 | C07C315/06<br>C07C317/04<br>H01M6/16<br>H01M10/40 |
| A | US 5 273 840 A (L.A. DOMINEY) 28. Dezember 1993 (1993-12-28) * Spalte 6, Zeile 1 - Zeile 30 * | 1 | |
| A | F.J. WALLER, ET AL.: "Tris(trifluoromethanesulphonyl)methide ("triflide") anion: convenient preparation, X-ray crystal structures, and exceptional catalytic activity as a counterion with ytterbium(III) and scandium(III)" JOURNAL OF ORGANIC CHEMISTRY, Bd. 64, Nr. 8, 26. März 1999 (1999-03-26), Seiten 2910-2913, XP002144668 American Chemical Society, Washington, DC, US ISSN: 0022-3263 * Seite 2912, rechte Spalte * | 1 | RECHERCHIERTE SACHGEBIETE (Int.Cl.7)<br>C07C<br>H01M |

-/--

Der vorliegende Recherchenbericht wurde für alle Patentansprüche erstellt

| Recherchenort | Abschlußdatum der Recherche | Prüfer |
|---|---|---|
| DEN HAAG | 10. August 2000 | English, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 00 10 8129

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
| D,A | L. TOROWSKY, ET AL.: "Tris((trifluoromethyl)sulphonyl)methane, HC(SO2CF3)3" INORGANIC CHEMISTRY, Bd. 27, Nr. 12, 15. Juni 1988 (1988-06-15), Seiten 2135-2137, XP000571907 American Chemical Society, Washington, DC, US ISSN: 0020-1669 * Seite 2136 * ----- | 1 | |
| | | | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| DEN HAAG | 10. August 2000 | English, R |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 00 10 8129

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-08-2000

| Im Recherchenbericht angeführtes Patentdokument | Datum der Veröffentlichung | Mitglied(er) der Patentfamilie | Datum der Veröffentlichung |
|---|---|---|---|
| US 5273840 A | 28-12-1993 | CA 2067751 A,C | 02-02-1992 |
| | | DE 69109847 D | 22-06-1995 |
| | | DE 69109847 T | 07-12-1995 |
| | | EP 0495073 A | 22-07-1992 |
| | | IL 98959 A | 14-05-1996 |
| | | JP 5503808 T | 17-06-1993 |
| | | WO 9202966 A | 20-02-1992 |

EPO FORM P0461

Für nähere Einzelheiten zu diesem Anhang : siehe Amtsblatt des Europäischen Patentamts, Nr.12/82